(19) Europäisches Patentamt
European Patent Office
Office européen des brevets

(11) Veröffentlichungsnummer: **0 317 912 B1**

(12) # EUROPÄISCHE PATENTSCHRIFT

(45) Veröffentlichungstag der Patentschrift: **02.02.94**

(51) Int. Cl.⁵: **C07D 231/16**, C07D 231/12, C07D 277/32, C07D 277/74, C07D 277/68

(21) Anmeldenummer: **88119195.1**

(22) Anmeldetag: **18.11.88**

(54) **Verfahren zur Herstellung von 1-Thiocyanatomethylethern.**

(30) Priorität: **27.11.87 DE 3740285**

(43) Veröffentlichungstag der Anmeldung:
**31.05.89 Patentblatt 89/22**

(45) Bekanntmachung des Hinweises auf die Patenterteilung:
**02.02.94 Patentblatt 94/05**

(84) Benannte Vertragsstaaten:
**AT BE CH DE ES FR GB GR IT LI NL SE**

(56) Entgegenhaltungen:
**US-A- 4 087 450**
**US-A- 4 101 546**

(73) Patentinhaber: **BASF Aktiengesellschaft**
**Carl-Bosch-Strasse 38**
**D-67063 Ludwigshafen(DE)**

(72) Erfinder: **Baus, Ulf, Dr.**
**Keltenweg 10**
**D-6915 Dossenheim(DE)**
Erfinder: **Reuther, Wolfgang, Dr.**
**Am Pferchelhang 16**
**D-6900 Heidelberg(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 317 912 B1

## Beschreibung

Die vorliegende Erfindung betrifft ein neues Verfahren zur Herstellung von 1-Thiocyanatomethylethern durch Umsetzung der entsprechenden Hydroxyverbindung mit Methylendirhodanid.

1-Thiocyanatomethylether und 1-Thiocyanatomethylthioether sind als biologisch aktive Stoffe bekannt. So sind z. B. 1-Thiocyanatomethylthiopyrazine fungizid wirksam (US-4101546). 1-Thiocyanatomethyl-2'-mercaptobenzthiazole (JP 60/132971) und 1-Thiocyanatomethyl-aryloxiether (US 4087450) sind bakterizid wirksam.

Die Herstellung der Verbindungen kann dabei in bekannter Weise auf verschiedenen Wegen erfolgen:

1. Die Hydroxygruppe wird metalliert und anschließend mit Halogenmethylthiocyanat umgesetzt (US-4101546), wobei anstelle einer Metallierung auch die Verwendung von Aminbasen und die Reaktionsführung in Gegenwart von Phasentransferkatalysatoren beschrieben ist.
2. Die Hydroxygruppe wird zunächst methyliert, der entstandene Methylether dann halogeniert und der Halogenmethylether mit einem Alkalithiocyanat umgesetzt.

Bei beiden Verfahren entsteht als Nebenprodukt in stöchiometrischen Mengen Alkalihalogenid, bzw. das Halogenid der Aminbase, das entsorgt werden muß. Das 2. o. g. Verfahren ist ein mehrstufiges Verfahren, das in der Regel zu hohen Ausbeuteverlusten führt.

Es bestand deshalb die Aufgabe, ein Verfahren zur Herstellung von 1-Thiocyanatomethylethern zu entwickeln, das die genannten Nachteile nicht hat.

Es wurde nun gefunden, daß man einen 1-Thiocyanatomethylether der allgemeinen Formel I

$R\text{-}X\text{-}CH_2SCN$ (I),

in der

R einen 5- oder 6-gliedrigen Ring bedeutet, der aromatisch oder nicht aromatisch sein kann und die Heteroatome O, S, N enthalten kann, einen durch Halogen substituierten Pyrazolrest oder durch Halogen substituierten Phenylrest oder einen Benzothiazolrest, und

X O oder S bedeutet,

erhält, wenn man ein Alkoholat der Formel II R-X-M, in der R und X die oben angegebenen Bedeutungen haben und M ein Alkali- oder Erdalkaliatom ist, mit Methylendirhodanid $CH_2(SCN)_2$ in einem inerten Lösungsmittel bei einer Temperatur von 0 bis 100 °C umsetzt.

Ein Alkali- oder Erdalkaliatom ist beispielsweise Natrium, Kalium, Magnesium.

R bedeutet beispielsweise einen 5-gliedrigen aromatischen Ring mit Stickstoffatomen, z.B. zwei Stickstoffatomen im Ring, insbesondere einen Pyrazolrest, der gegebenenfalls durch Halogen, z.B. durch Chlor substituiert ist, beispielsweise den 4-Chlorpyrazolrest. R bedeutet ferner einen substituierten 5-gliedrigen aromatischen S und N enthaltenden Rest, beispielsweise den Thiazolrest, insbesondere den Benzothiazolrest. R bedeutet auch einen Phenylrest, der gegebenenfalls durch Halogen, z.B. Chlor, beispielsweise zweifach durch Chlor substituiert ist, insbesondere den 2,4-Dichlorphenylrest.

Die Umsetzung erfolgt beispielsweise mit einem überschuß (z.B. bis zu 10 % überschuß) an einem der beiden Reaktionsteilnehmer. Bevorzugt wird die Umsetzung stöchiometrischer Mengen. Die Umsetzung erfolgt beispielsweise bei einer Temperatur von 0 bis 100 °C. Bevorzugt wird 10 bis 70 °C, insbesondere Raumtemperatur (20 °C).

Die Umsetzung erfolgt beispielsweise in einem inerten Lösungsmittel, z.B. einem Ether (Diethylether) oder einem aromatischen Kohlenwasserstoff (Toluol) oder einem cyclischen Ether (Tetrahydrofuran). Die Abtrennung des Endprodukts von der Reaktionsmischung nach Beendigung der Umsetzung kann in üblicher Weise erfolgen, z.B. durch Verteilen der Reaktionsmischung in einem Zwei-Phasensystem zwischen Wasser und einem organischen Lösungsmittel und Abtrennung der organischen Phase, die das Endprodukt enthält, während die Alkali- oder Erdalkalithiocyanate, die bei der Umsetzung entstanden sind, in der wäßrigen Phase gelöst sind und wieder zur Herstellung von Methylendirhodanid verwendet werden können (vgl. JP 51/6928).

Auf diese Weise wird vermieden, daß das Abwasser durch Abfallprodukte belastet wird.

Das Alkoholat der Formel II R-X-M erhält man durch Umsetzung des Alkohols der Formel I R-X-H mit einer Alkali- oder Erdalkaliverbindung, z.B. einer Natrium-, Kalium- oder Magnesiumverbindung, insbesondere einer alkalischen Verbindung, beispielsweise Alkalihydroxid, Alkalihydrid, Alkalicarbonat, z.B. NaOH, NaH oder $Na_2CO_3$.

Die Herstellung der Verbindungen der allgemeinen Formel I nach dem erfindungsgemäßen Verfahren sei an folgendem Beispiel erläutert:

Beispiel 1

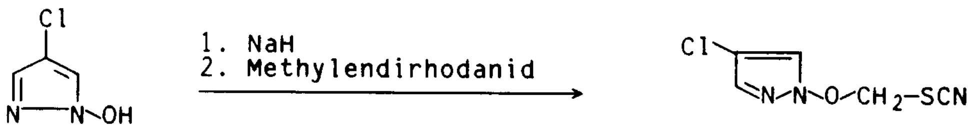


5,93 g (50 mmol) 4-Chlor-N-Hydroxipyrazol (DE-32 05 456) werden bei Raumtemperatur in Tetrahydrofuran mit 1,5 g (50 mmol) einer 80 %igen (Gew.%) Suspension von NaH in Paraffinöl versetzt. Nach beendeter Wasserstoffentwicklung wird die Suspension zu einer Lösung von 6,5 g (50 mmol) Methylendirhodanid (in Tetrahydrofuran) getropft und die Reaktionsmischung noch 4 h bei Raumtemperatur gerührt.

Zur Aufarbeitung gießt man die Mischung in $H_2O$/Ether (1:1), trennt die wässrige Phase ab, extrahiert mehrmals mit Ether und trocknet die vereinten org. Phasen mit $Na_2SO_4$. Nach Entfernen des Lösemittels im Vakuum wird ein HNMR-spektroskopisch und elementaranalytisch reines Rohprodukt erhalten. Ausbeute: 7,8 g (82 %) 4-Chlor-1-[(thiocyanato)-methoxy]-pyrazol, Fp 60 °C.

| | | | | | | |
|---|---|---|---|---|---|---|
| Elementaranalyse: | ber. | C 31,6 | H 2,1 | Cl 18,7 | N 22,1 | S 16,8 |
| | gef. | C 32,3 | H 2,4 | Cl 18,7 | N 21,5 | S 16,7 |

Weitere Beispiele:

| Bsp.-Nr. | Struktur | Ausbeute | | C | H | N | Hal | S |
|---|---|---|---|---|---|---|---|---|
| 2 | 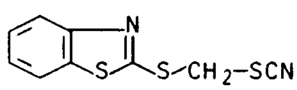 | 95 % | ber. | 45,3 | 2,5 | 11,7 | - | 40,3 |
| | | | gef. | 45,6 | 3,1 | 11,8 | - | 39,5 |

Die gemäß Beispiel 1 erhaltene Verbindung eignet sich zum Schutz von verschiedenen Materialien gegen die Zerstörung durch Bakterien oder Pilze oder gegen den Bewuchs durch Mikroorganismen. Solche Materialien sind z.B. Leime, Klebstoffe, Stärkelösungen, Wachsemulsionen, Kunststoffdispersionen, Dispersionsfarben, Textilien, Leder.

## Patentansprüche

**1.** Verfahren zur Herstellung von 1-Thiocyanatomethylethern der allgemeinen Formel I

$R\text{-}X\text{-}CH_2SCN$ (I)

in der

R einen 5- oder 6-gliedrigen Ring bedeutet, der aromatisch oder nicht aromatisch sein kann und die Heteroatome O, S, N enthalten kann, einen durch Halogen substituierten Pyrazolrest oder durch Halogen substituierten Phenylrest oder Benzothiazolrest, und

X O oder S bedeutet, dadurch gekennzeichnet, daß man ein Alkoholat der Formel II R-X-M, in der R und X die oben angegebenen Bedeutungen haben und M ein Alkali- oder Erdalkaliatom ist, mit Methylendirhodanid $CH_2(SCN)_2$ in einem inerten Lösungsmittel bei einer Temperatur von 0 bis 100 °C umsetzt.

**2.** Verfahren nach Anspruch 1, dadurch gekennzeichnet, daß man ein Alkoholat verwendet, das aus einem Alkohol der Formel III R-X-H, in der R und X die oben genannten Bedeutungen haben, mit einer Alkali- oder Erdalkaliverbindung hergestellt worden ist.

## Claims

1. A process for preparing 1-thiocyanatomethyl ethers of the general formula (I)

   $R\text{-}X\text{-}CH_2SCN$ (I),

   where
   R is a 5- or 6-membered ring, which can be aromatic or nonaromatic and may contain the hetero atoms O, S and N, a halogen-substituted pyrazole radical or a halogen-substituted phenyl or benzothiazole radical, and
   X is O or S, which comprises reacting an alkoxide of the formula II R-X-M, where R and X are each as defined above and M is an alkali or alkaline earth metal atom, with methylene dithiocyanate $CH_2(SCN)_2$ in an inert solvent at from 0 to 100 °C.

2. A process as claimed in claim 1, wherein the alkoxide used has been prepared with an alkali or alkaline earth metal compound from an alcohol of the formula III R-X-H, where R and X are each as defined above.

## Revendications

1. Procédé de préparation d'éthers 1-thiocyanatométhyliques de formule générale I

   $R\text{-}X\text{-}CH_2SCN$ (I),

   dans laquelle
   R représente un cycle à 5 ou 6 chaînons qui peut être aromatique ou non et peut contenir les hétéroatomes O, S, N, un groupe pyrazole substitué par des halogènes ou un groupe phényle substitué par des nalogènes ou un groupe benzothiazole, et
   X représente O ou S,
   ce procédé se caractérisant en ce que l'on fait réagir un alcoolate de formule II, R-X-M, dans laquelle R et X ont les significations indiquées ci-dessus et M représente un atome de métal alcalin ou alcalino-terreux, avec du méthyléne-dithiocyanate $CH_2(SCN)_2$ dans un solvant inverte à une température de 0 à 100 °C.

2. Procédé selon la revendication 1, caractérisé en ce que l'on utilise un alcoolate qui a été préparé à partir d'un alcool de formule III, R-X-H, dans laquelle R et X ont les significations indiquées ci-dessus, et d'un dérivé de métal alcalin ou alcalino-terreux.